# FASCICULE DE BREVET EUROPEEN

(11) **EP 0 666 075 B1**
(45) Date de publication et mention de la délivrance du brevet: **07.04.1999**
(21) Numéro de dépôt: 95400043.6
(22) Date de dépôt: 10.01.1995
(51) Int. Cl.: A61K 7/42

(54) **Composition cosmétique ou dermatologique contenant une dispersion aqueuse de particules solides d'un homopolymère-filtre U.V.**
Eine kosmetische oder dermatologische Zusammensetzung enthaltend eine wässrige Dispersion von einer feinteiligen U.V. Homopolymerfiltersubstanz
Cosmetic or dermatologic composition containing an aqueous dispersion of solid particles of an U.V. screen homopolymer

(30) Priorité: 10.01.1994 FR 9400162
(43) Date de publication de la demande: 09.08.1995
(73) Titulaire: L'OREAL, 75008 Paris (FR)
(72) Inventeur: Mondet, Jean, F-93700 Drancy (FR); Mougin, Nathalie, F-75011 Paris (FR)
(74) Mandataire: Stalla-Bourdillon, Bernard

(56) Documents cités:
- FR-A- 2 601 365
- FR-A- 2 680 684
- GB-A- 2 170 105
- DATABASE WPI Section Ch, Week 5092 Derwent Publications Ltd., London, GB; Class A09, AN 92-415716 & WO-A-92 20721 (KAO CORP.) , 26 Novembre 1992
- PATENT ABSTRACTS OF JAPAN vol. 13 no. 199 (C-594) ,11 Mai 1989 & JP-A-01 020201 (DAINIPPON INK. & CHEM. INC.) 24 Janvier 1989,
- DATABASE WPI Section Ch, Week 1693 Derwent Publications Ltd., London, GB; Class A04, AN 93-129127 & JP-A-05 065 316 (TOKYO KEIKAKU KK) , 19 Mars 1993

## Description

La présente invention a pour objet une composition cosmétique ou dermatologique destinée à protéger la peau ou un substrat kératinique contre les effets indésirés des radiations ultra-violettes, contenant une dispersion aqueuse de particules solides d'un homopolymère-filtre.

On sait que les radiations ultra-violettes ayant une longueur d'onde dans la zone comprise entre 280 et 315 nm environ, sont responsables des inflammations ou érythèmes de la peau observés lorsque le corps humain est soumis aux rayons solaires sans protection. Cette zone de longueurs d'onde est parfois appelée "zone érythémateuse".

On sait également que les radiations ultra-violettes responsables du bronzage de la peau ont une longueur d'onde comprise entre 315 et 400 nm environ.

On connaît de nombreux composés organiques qui ont la propriété d'absorber l'ultra-violet (U.V.) dans la zone érythémateuse tout en laissant passer les radiations responsables du bronzage et on a déjà proposé l'utilisation de tels composés dits "filtres solaires" dans des compositions cosmétiques favorisant le bronzage de la peau tout en évitant les brûlures et les irritations de la peau.

D'autre part, pour les personnes particulièrement sensibles aux effets du soleil et souhaitant éviter tout bronzage on utilise, dans les compositions cosmétiques, des filtres solaires dits "écran total" formant une barrière plus ou moins importante aux radiations ultra-violettes dans la zone de longueurs d'onde comprise entre 315-400 nm éventuellement en association avec des composés filtrant les radiations ultra-violettes dans la zone érythémateuse, de façon à éviter à la fois le bronzage et les brûlures de la peau.

Il a été proposé, notamment dans FR 73.23254 et 76.23174, l'utilisation de composés-filtres solaires liés par covalence à des polymères. La présentation sous forme de polymères antisolaires présente l'avantage de réduire ou de supprimer la pénétration du composé-filtre dans l'organisme au travers de l'épiderme, ce qui permet d'éviter tout risque d'effets secondaires toxiques.

Toutefois, la poursuite de l'étude de tels "polymères antisolaires" ou "polymères-filtres" a montré que leur utilisation présentait certains inconvénients.

En effet, pour obtenir des compositions ayant un pouvoir filtrant élevé, une des solutions envisagées est d'utiliser des concentrations importantes en polymère antisolaire. Ceci soulève cependant d'importantes difficultés tant au stade de la préparation des compositions cosmétiques qu'à celui de l'application de ces compositions sur la peau.

Ainsi, pour des concentrations élevées, la mise en solution des polymères antisolaires est difficile, ce qui constitue un inconvénient au niveau de la formulation. En outre, de telles compositions ne sont pas jugées cosmétiquement satisfaisantes par les utilisateurs car elles présentent un toucher collant et gras qui est désagréable.

Si en vue d'améliorer le pouvoir filtrant, il est possible d'utiliser des polymères du type homopolymère dont chaque motif est substitué par un composé-filtre, on observe néanmoins les mêmes inconvénients que décrits précédemment, quant aux propriétés cosmétiques.

De tels polymères du type homopolymère ont été décrits dans FR 86 00 274 pour la réalisation de compositions cosmétiques notamment huileuses.

Par ailleurs, pour obtenir des compositions présentant une rémanence suffisante, il est préférable que le polymère-filtre soit insoluble à l'eau, ce qui complique par conséquent considérablement la formulation de ces compositions.

Afin de remédier à ces différents inconvénients, il a été proposé d'utiliser des latex de copolymères-filtres.

Par l'expression "latex", on désigne une dispersion de particules solides d'un polymère, celles-ci étant obtenues directement par polymérisation d'un ou plusieurs monomères dans une phase aqueuse appropriée.

Ainsi, il a été décrit dans JP-50-65316, la réalisation de latex résultant de la copolymérisation en émulsion aqueuse de méthacrylate de méthyle, d'un monomère bifonctionnel et d'un comonomère à groupement filtre.

Cependant, les latex à base de ces copolymères-filtres présentent un pouvoir filtrant réduit. De plus, la préparation de ces latex conduit à la formation soit de copolymères-filtres présentant des poids moléculaires relativement élevés et donc difficilement formulables, soit de copolymères-filtres de poids moléculaires plus faibles mais nécessitant l'incorporation de quantités très importantes d'agent de transfert les rendant extrêmement difficiles à purifier.

Il a par ailleurs été proposé l'utilisation de pseudo-latex d'un copolymère-filtre.

Par l'expression "pseudo-latex", on désigne une dispersion de particules solides d'un polymère, celles-ci étant obtenues par dissolution d'un polymère dans un solvant organique, dispersion de la solution dans l'eau puis évaporation dudit solvant.

Ainsi, il a été décrit dans WO-92.20721, l'utilisation d'un pseudo-latex constitué de particules d'un copolymère radicalaire comprenant, outre les motifs portant les groupements filtres solaires, des motifs à groupements salifiables.

Toutefois, les compositions obtenues à partir de tels pseudo-latex présentent un pouvoir filtrant relativement faible et une rémanence à l'eau très insuffisante.

On a maintenant constaté de façon surprenante et inattendue qu'il était possible de réaliser des compositions cosmétiques ou dermatologiques, rémanentes à l'eau, aisément formulables, et ayant un excellent pouvoir filtrant, en utilisant des dispersions aqueuses de particules solides d'homopolymères-filtres.

La présente invention a donc pour objet une composition cosmétique ou dermatologique destinée à protéger la peau ou un substrat kératinique contre les effets indésirés des radiations ultra-violettes, contenant au moins une dispersion aqueuse de particules solides, de diamètre moyen compris entre 10 et 400 nm, d'un homopolymère-filtre constitué de motifs répétitifs de formule suivante : 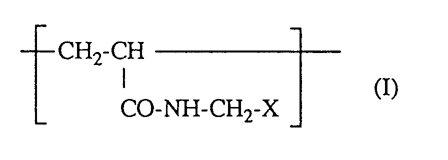 dans laquelle :
X représente un groupement aromatique présentant une absorption ultra-violette dans la zone de longueurs d'onde comprise entre 280 et 400 nm.

Parmi les homopolymères constitués des motifs de formule (I), on utilise, de préférence, selon l'invention, ceux dans lesquels X est choisi parmi les groupements de formules : 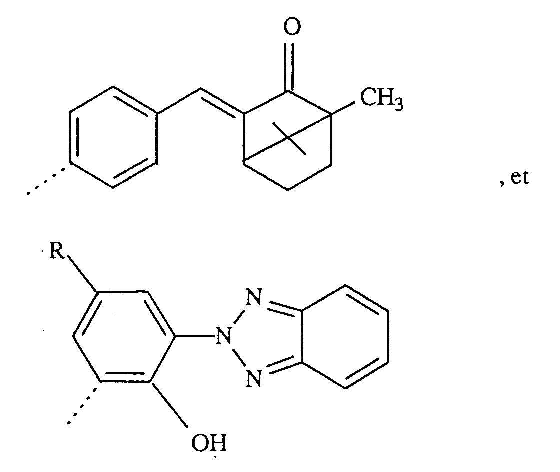 R représentant un groupement tertio-octyle, c'est-à-dire ceux pour lesquels X représente respectivement les restes du benzylidène-3 d1 camphre et de l'[(hydroxy-2' tert-octyl-5' phényl)-2] 2H benzotriazole.

De façon particulièrement préférée, X représente le reste du benzylidène-3 d1 camphre.

Selon un mode de réalisation particulièrement préféré des compositions selon l'invention, l'homopolymère-filtre constituant les particules solides de la dispersion aqueuse a un poids moléculaire inférieur ou égal à 20.000 et de préférence compris entre 1.000 et 17.000.

Ce poids moléculaire est déterminé au sommet du pic de chromatographie par exclusion stérique, par le tétrahydrofuranne, en comparaison avec des échantillons-étalons de polystyrène.

Dans les compositions selon l'invention, la dispersion aqueuse de particules solides d'homopolymère-filtre telle que définie précédemment est de préférence présente en une proportion telle que la concentration en homopolymère-filtre soit comprise entre 0,5 et 15 % en poids par rapport au poids total de la composition.

Parmi les dispersions aqueuses de particules solides d'homopolymère-filtre, pouvant être utilisées selon l'invention, on peut citer notamment des latex et des pseudo-latex.

Selon un mode de réalisation préféré, on utilise dans les compositions selon l'invention, des dispersions aqueuses sous forme de pseudo-latex.

Les pseudo-latex d'homopolymère-filtre tels que définis précédemment sont obtenus selon les méthodes connues de préparation des pseudo-latex.

Le procédé général de préparation des pseudo-latex consiste :
(i) à dissoudre l'homopolymère-filtre, insoluble dans l'eau, dans un solvant organique, miscible ou partiellement miscible dans l'eau,
(ii) à disperser sous agitation la solution ainsi obtenue dans de l'eau à une température comprise entre la température ambiante et 70°C environ,
(iii) à homogénéiser la suspension ainsi obtenue, et
(iv) à évaporer sous pression réduite, et de préférence sous léger chauffage, le solvant organique jusqu'à son élimination totale.

On obtient ainsi un pseudo-latex, c'est-à-dire une dispersion aqueuse de particules solides, celles-ci ayant généralement une taille inférieure au µm. La taille moyenne des particules du pseudo-latex ainsi obtenu, et utilisé dans les compositions selon l'invention, est généralement comprise entre 10 et 400 nm et de préférence comprise entre 20 et 350 nm.

La proportion en homopolymère-filtre dans les pseudo-latex pouvant être utilisés dans les compositions selon l'invention est généralement comprise entre 1 et 40 % et de préférence entre 10 et 30 % en poids par rapport au poids total du pseudo-latex.

Le solvant organique utilisé doit être un solvant volatil ou un mélange de tels solvants présentant un point d'ébullition inférieur à celui de l'eau. Parmi ceux-ci, on peut citer notamment l'acétone, la méthyléthylcétone, le tétrahydrofuranne, le 1,2-dichloroéthane, l'acétate de méthyle, l'acétate d'éthyle, l'isopropanol et l'éthanol.

On peut en outre ajouter à la phase organique, un agent anti-mousse de type classique, celui-ci permettant de faciliter l'étape d'évaporation du solvant organique.

Selon le procédé général décrit précédemment, on utilise également de préférence, un agent dispersant choisi parmi un agent tensio-actif, un mélange d'agents tensio-actifs ou un polymère colloïde protecteur ou encore un mélange agent tensio-actif/polymère colloïde protecteur, en vue d'améliorer la stabilisation des particules.

Les agents tensio-actifs utilisables selon l'invention peuvent être du type anionique, non-ionique ou cationique. On utilise toutefois de préférence des agents tensio-actifs de type anionique ou non-ionique. Parmi ceux-ci, on peut citer notamment le laurylsulfate de sodium.

On peut en outre associer à l'agent tensio-actif utilisé un costabilisant soluble dans la phase organique tel que l'alcool cétylique.

Comme polymères colloïdes protecteurs, on peut citer notamment les éthers de cellulose hydrosolubles, l'alcool polyvinylique et la gomme arabique.

De plus, on peut introduire dans les pseudo-latex utilisés dans les compositions selon l'invention, en vue d'améliorer leur propriétés cosmétiques et mécaniques, un agent plastifiant en une proportion comprise entre 5 et 40 % et de préférence entre 10 et 30 % en poids par rapport au poids de l'homopolymère-filtre, ledit agent se distribuant selon son coefficient de partage entre les particules et la phase aqueuse du pseudo-latex.

L'agent plastifiant qui peut être du type hydrophile ou hydrophobe, est de préférence introduit en mélange dans le solvant organique lors de la préparation du pseudo-latex, et notamment lorsqu'il est du type hydrophobe.

Lorsque l'agent plastifiant est du type hydrophile, il peut être introduit après formation du pseudo-latex dans la phase aqueuse.

Parmi les agents plastifiants pouvant être utilisés dans les compositions selon l'invention, on peut citer :
- les "Carbitols®" de la Société Union Carbide à savoir le "Carbitol®" ou diéthylène glycol éthyléther, le "méthyl Carbitol®" ou diéthylène glycol méthyléther, le "butyl Carbitol®" ou diéthylène glycol butyléther ou encore l'"hexyl Carbitol®" ou diéthylène glycol hexyléther,
- les "Cellosolves®" de la Société Union Carbide à savoir le "Cellosolve®" ou éthylène glycol éthyléther, le "butyl Cellosolve®" ou éthylène glycol butyléther, l'"hexyl Cellosolve®" ou éthylène glycol hexyléther,
- les dérivés de propylène glycol et en particulier le propylène glycol phényléther, le propylène glycol diacétate, le dipropylène glycol butyléther, le tripropylène glycol butyléther, ainsi que les "Dowanols®" de la Société Dow Chemical à savoir le "Dowanol PM®" ou propylène glycol méthyléther, le "Dowanol DPM®" ou dipropylène glycol méthyléther et le "Dowanol TPM®" ou tripropylène glycol méthyléther.

On peut encore citer :
- le diéthylène glycol méthyléther ou "Dowanol DM®" de la Société Dow Chemical,
- l'huile de ricin oxyéthylénée à 40 moles d'oxyde d'éthylène telle que celle vendue par la Société Rhône Poulenc sous la dénomination de "Mulgofen EL-719®",
- l'alcool benzylique,
- le citrate de triéthyle vendu par la Société Pfizer sous la dénomination de "Citroflex-2®",
- le 1,3-butylène glycol,
- les phtalates et adipates de diéthyle, de dibutyle et de diisopropyle,
- les tartrates de diéthyle et de dibutyle,
- les phosphates de diéthyle, de dibutyle et de diéthyl-2 hexyle, et
- les esters de glycérol tels que le diacétate de glycérol (diacétine) et le macétate de glycérol (triacétine).

On utilise de préférence un agent plastifiant choisi dans le groupe constitué par le dipropylène glycol méthyléther, le tripropylène glycol méthyléther, l'adipate de diéthyle et l'adipate de diisopropyle.

La composition cosmétique ou dermatologique selon l'invention peut être utilisée comme composition protectrice de l'épiderme humain ou des cheveux contre les radiations ultra-violettes, sous forme d'une composition antisolaire ou d'un produit de maquillage.

La composition cosmétique ou dermatologique est de préférence une solution aqueuse ou hydroalcoolique, une lotion, une lotion épaissie, un gel, un lait, une crème, une émulsion, une dispersion vésiculaire ou éventuellement être conditionnée en aérosol et se présenter sous forme d'une mousse ou d'un spray.

Elle peut contenir les adjuvants cosmétiques ou dermatologiques habituellement utilisés tels que des corps gras, des silicones, des agents épaississants, des agents adoucissants, des filtres solaires UV-A, UV-B, ou à bande large, des agents anti-mousse, des agents hydratants, des parfums, des conservateurs, des agents tensioactifs, des agents plastifiants, des charges, des agents séquestrants, des polymères anioniques, cationiques, non-ioniques, amphotères, ou leurs mélanges, des propulseurs, des agents alcalinisants ou acidifiants, des colorants, des pigments d'oxydes métalliques ou tout autre ingrédient habituellement utilisé en cosmétique ou en dermatologie

Parmi les solvants organiques, on peut citer les alcools et polyols inférieurs tels que l'éthanol, l'isopropanol, le propylène glycol, la glycérine et le sorbitol. Ceux-ci sont alors utilisés en une proportion telle qu'ils ne peuvent solubiliser les particules solides de l'homopolymère-filtre dans la composition.

Les corps gras peuvent être constitués par une huile ou une cire ou leur mélange, par des acides gras, des esters d'acides gras, des alcools gras, de la vaseline, de la paraffine, de la lanoline, de la lanoline hydrogénée, et de la lanoline acétylée.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin, les huiles de silicone et les isoparaffines.

Les cires sont choisies parmi les cires animales, fossiles, végétales, minérales ou de synthèse. On peut citer notamment la cire d'abeilles, la cire de carnauba, la cire de candelilla, la cire de canne à sucre, la cire du Japon, l'ozokérite, la cire de Montan, les cires microcristallines, les paraffines, les cires et résines de silicone.

Les esters d'acides gras sont par exemple le myristate d'isopropyle, l'adipate d'isopropyle, le palmitate d'isopropyle, le palmitate d'octyle, les benzoates d'alcools gras en C₁₂-C₁₆ tel que celui commercialisé sous la dénomination de "Finsolv TN®" par la Société Finetex, l'alcool myristique oxypropyléné à 3 moles d'oxyde de propylène tel que celui commercialisé sous la dénomination de "Witconol APM®" par la Société Witco, les triglycérides d'acides caprique et caprylique tel que le mélange commercialisé sous la dénomination de "Miglyol 812®" par la Société Huls.

On peut également introduire dans la composition cosmétique ou dermatologique selon l'invention des agents épaississants connus de l'état de la technique qui peuvent être choisis parmi les polymères d'acide acrylique, réticulés ou non, et particulièrement les acides polyacryliques réticulés par un agent polyfonctionnel tels que les produits commercialisés sous la dénomination de "Carbopol®" par la Société Goodrich, les dérivés de cellulose tels que la méthylcellulose, l' hydroxyéthylcellulose, l'hydroxypropylméthylcellulose, les sels de sodium de la carboxyméthylcellulose, et les mélanges d'alcool cétylstéarylique et d'alcool cétylstéarylique oxyéthyléné à 33 moles d'oxyde d'éthylène.

Selon un mode de réalisation préféré de la composition selon l'invention, celle-ci se présente sous forme d'une émulsion, dont la phase aqueuse est constituée notamment par la dispersion aqueuse de particules solides telle que définie ci-dessus et la phase grasse contient un homopolymère-filtre de formule (I) sous forme solubilisée.

On observe avec ce type de composition une potentialisation du pouvoir filtrant.

Selon un mode de réalisation particulièrement préféré, l'homopolymère de formule (I) utilisé décrit précédemment est l'homopolymère de l'[(acrylamidométhyl-4'-benzylidène)-3]d1camphre.

La quantité en homopolymère-filtre de formule (I) présente dans la phase grasse doit être telle que la proportion totale en homopolymère-filtre de formule (I) dans la composition ne soit pas supérieure à 15 % en poids par rapport au poids total de la composition.

L'émulsion peut contenir en outre des agents tensioactifs anioniques, non-ioniques, cationiques ou amphotères.

Selon un autre mode de réalisation des compositions selon l'invention, celles-ci se présentent sous forme de dispersions vésiculaires de lipides amphiphiles ioniques ou non-ioniques, préparées selon des procédés connus, comme par exemple en faisant gonfler les lipides dans une solution aqueuse pour former des sphérules dispersées dans un milieu aqueux, comme décrit dans l'article Bangham, Standish & Watkins, J. Mol. Biol. 13, 238 (1965) ou dans FR-2.315.991 et 2.416.008. Dans cette forme de réalisation des compositions selon l'invention, la solution aqueuse contient bien entendu une dispersion aqueuse de particules solides telle que définie précédemment. Les vésicules lipidiques peuvent éventuellement comprendre un homopolymère-filtre de formule (I) tel que défini précédemment, celui-ci étant toutefois sous forme solubilisée.

Lorsque la composition cosmétique ou dermatologique selon l'invention est utilisée pour la protection de l'épiderme humain contre les radiations ultra-violettes, ou pour prévenir les photodermatites et les lucites, elle peut se présenter sous forme d'une lotion aqueuse ou hydroalcoolique, ou encore sous forme d'une émulsion telle qu'une crème ou un lait, sous forme d'une pommade, d'un gel ou d'une mousse aérosol.

Lorsque la composition cosmétique selon l'invention est destinée à protéger des radiations ultra-violettes les cheveux naturels ou sensibilisés, la composition peut se présenter par exemple sous forme d'une lotion, d'un gel, d'une émulsion, d'un spray de coiffage ou d'une laque.

On va maintenant donner à titre d'illustration, plusieurs exemples de préparation de pseudo-latex pouvant être utilisés selon l'invention ainsi que de compositions cosmétiques les contenant.

### EXEMPLES DE PREPARATION DE PSEUDO-LATEX

### EXEMPLE I : Préparation du pseudo-latex à base de l'homopolymère de l'[(acrylamidométhyl-4'-benzylidène)-3]camphre

La préparation de cet homopolymère-filtre est décrite à l'exemple 1 de FR 86.00274 (2.597.336) et se présente sous forme d'une poudre jaune clair.

70 g de l'homopolymère-filtre défini ci-dessus sont additionnés petit-à-petit sous agitation à une solution homogène de 1.050 g de 1,2-dichloroéthane et de 1,75 g d'alcool cétylique, formant ainsi une phase organique.

La phase organique ainsi obtenue est alors ajoutée en 30 min sous agitation à 3.000 tr/min, à l'aide d'un disperseur de type Moritz, et à température ambiante, à une solution constituée de 875 g d'eau permutée et de 1,75 g de laurylsulfate de sodium.

Après la fin de l'addition de la phase organique, on poursuit l'agitation à 3.000 tr/min pendant environ 30 min, à température ambiante, ce qui permet de conduire à l'obtention d'une émulsion relativement grossière. On procède alors à l'homogénéisation de l'émulsion à l'aide d'un homogénéiseur haute pression du type Soavib et Figli, modèle OBL n° 2032, sous une pression de 75.10⁶ Pa. Après trois passages, on obtient une émulsion fine et homogène.

On procède alors à la concentration à l'aide d'un évaporateur rotatif sous vide partiel à une température inférieure ou égale à 50°C. Après élimination totale du 1,2-dichloroéthane, on obtient une dispersion stable dont la concentration en homopolymère-filtre est de 19 % en poids par rapport au poids total de la dispersion.

La taille des particules a été mesurée en diffusion quasi-élastique de lumière au Coulter modèle M4 et a donné les résultats suivants :
- Taille moyenne des particules : 133 nm
- Facteur de polydispersité : 0,12.

### EXEMPLE II : Préparation du pseudo-latex à base de l'homopolymère de l'[(acrylamidométhyl-4'-benzylidène)-3]camphre plastifié à 30 % en poids par rapport au poids total du polymère

Selon un mode opératoire analogue à celui décrit à l'exemple I, on prépare à température ambiante, un pseudo-latex à partir d'une phase organique constituée de 70 g d'homopolymère de l'[(acrylamidométhyl-4'-benzylidène)-3]camphre tel que préparé selon l'exemple 1 de FR 86.00274, 1.050 g de 1,2-dichloroéthane, 1,75 g d'alcool cétylique et de 21 g d'adipate de diéthyle. On obtient ainsi une dispersion stable, homogène et plastifiée dont la concentration en homopolymère-filtre est de 18 % en poids par rapport au poids total de la dispersion.

La taille des particules a été mesurée en diffusion quasi-élastique de lumière au Coulter modèle M4 et a donné les résultats suivants :
- Taille moyenne des particules : 127 nm
- Facteur de polydispersité : 0,13.

### EXEMPLE III : Préparation du pseudo-latex à base de l'homopolymère d' [(hydroxy-2' acrylamidométhyl-3' t-octyl-5'phényl)-2]2H-benzotriazole.

La préparation de l'homopolymère est décrite à l'exemple 3 de FR 86.00274 (2.597.336).

Selon un mode opératoire identique à celui décrit à l'exemple I, on prépare un pseudo-latex à partir de 70 g de l'homopolymère-filtre défini ci-dessus.

On obtient ainsi une dispersion aqueuse de particules solides sous forme de pseudo-latex dont la concentration en homopolymère-filtre est de 16 % en poids par rapport au poids total de la dispersion.

La taille des particules a été mesurée en diffusion quasi-élastique de lumière au Coulter modèle M4 et a donné les résultats suivants :
- Taille moyenne des particules : 141 nm
- Facteur de polydispersité : < 0,1.

### EXEMPLES DE COMPOSITIONS COSMETIQUES

### EXEMPLE 1 : Crème anti-solaire

| *Phase A :* | |
|---|---|
| - Composition auto-émulsionnable commercialisée sous la dénomination de "Montanov 68®" par la Société Seppic | 5 g |
| - Huile de silicone commercialisée sous la dénomination de "DC 245 Fluid®" par la Société Dow Corning | 20 g |

| *Phase B :* | |
|---|---|
| - Pseudo-latex de l'exemple I | 24 g |
| - Glycérine | 3 g |
| - Conservateurs qs | |
| - Eau q.s.p. | 100 g |

Cette crème est obtenue en portant séparément les phases A et B à 80°C. On ajoute ensuite sous agitation la phase A à la phase B. On poursuit alors l'agitation jusqu'à refroidissement du mélange à la température ambiante.

On obtient ainsi une crème anti-solaire sous forme d'une émulsion huile-dans-eau, stable et homogène.

Cette crème s'applique facilement sur la peau, lui confère une bonne protection vis-à-vis des radiations ultra-violettes et résiste à l'eau.

### EXEMPLE 2 : Crème anti-solaire

Selon le même mode opératoire que décrit à l'exemple 1, on prépare une crème anti-solaire ayant la composition suivante :

| *Phase A :* | |
|---|---|
| - Composition auto-émulsionnable commercialisée sous la dénomination de "Montanov 68®" par la Société Seppic | 5,0 g |
| - Huile de silicone commercialisée sous la dénomination de "DC 245 Fluid®" par la Société Dow Corning | 20,0 g |

| *Phase B :* | |
|---|---|
| - Pseudo-latex de l'exemple II | 34,2 g |
| - Glycérine | 3,0 g |
| - Eau q.s.p. | 100 g |

### EXEMPLE 3 : Crème anti-solaire

Selon le même mode opératoire que décrit a' l'exemple 1, on prépare une crème anti-solaire ayant la composition suivante :

| *Phase A :* | |
|---|---|
| - Composition auto-émulsionnable commercialisée sous la dénomination de "Montanov 68®" par la Société Seppic | 5,0 g |
| - Huile de silicone commercialisée sous la dénomination de "DC 245 Fluid®" par la Société Dow Corning | 20,0 g |

| *Phase B :* | |
|---|---|
| - Pseudo-latex de l'exemple III | 19,0 g |
| - Glycérine | 3,0 g |
| - Eau q.s.p. | 100 g |

### EXEMPLE 4 : Crème anti-solaire

Selon le même mode opératoire que décrit à l'exemple 1, on prépare une crème anti-solaire ayant la composition suivante :

| *Phase A :* | |
|---|---|
| - Mélange d'alcool cétylstéarylique et d'alcool cétylstéarylique oxyéthyléné à 33 moles d'oxyde d'éthylène (80/20) commercialisé sous la dénomination de "Sinnowax AO®" par la Société Henkel | 8 g |
| - Mélange de mono- et di-stéarate de glycérol commercialisé sous la dénomination "Géléol copeaux®" par la Société Gattefosse | 2 g |
| - Alcool cétylique | 1,5 g |
| - Huile de silicone commercialisée sous la dénomination de "Silbione 10 047 V 300®" par la Société Rhône Poulenc | 15 g |

| *Phase B :* | |
|---|---|
| - Pseudolatex de l'exemple I | 26,3 g |
| - Glycérine | 15 g |
| - Conservateurs qs | |
| - Eau q.s.p. | 100 g |

### EXEMPLE 5 : Crème de jour

| *Phase A :* | |
|---|---|
| - Ether de 2-éthyl hexyle et de palmitate de glycéryle commercialisé sous la dénomination de "Mexanyl GP®" par la Société Chimex | 12,0 g |
| - Isostéarate de glycéryle | 8,0 g |
| - Mélange de stéarate de glycéryle et de monostéarate de polyéthylèneglycol à 100 moles d'oxyde d'éthylène commercialisé sous la dénomination de "Arlacel 165®" par la Société ICI | 2,5 g |
| - Ether d'alcool stéarylique et de polyéthylèneglycol à 2 moles d'oxyde d'éthylène commercialisé sous la dénomination de "Brij 72®" par la Société ICI | 2,0 g |
| - Alcool cétylique | 1,5 g |
| - Parfum | 0,6 g |

| *Phase B :* | |
|---|---|
| - Pseudo-latex de l'exemple I | 26,3 g |
| - Conservateurs | 0,3 g |
| - Eau q.s.p. | 100 g |

Cette crème est obtenue en portant séparément les phases A et B à 70°C.

On ajoute ensuite sous agitation la phase A à la phase B. On poursuit alors l'agitation jusqu'à refroidissement du mélange à la température ambiance.

On obtient ainsi une crème de jour, sous forme d'une émulsion huile-dans-eau, épaisse et filmogène.

Cette crème qui s'étale facilement sur la peau, est adoucissante et résiste à l'eau.

De plus, elle présente un excellent indice de protection U.V.

### EXEMPLE 6 : Crème de soin

Selon le même mode opératoire que décrit à l'exemple 5, on prépare une crème ayant la composition suivante :

| *Phase A :* | |
|---|---|
| - Cyclométhicone | 5,0 g |
| - Huile de vaseline | 10,0 g |
| - Mélange d'octanoate de cétéaryle et de myristate d'isopropyle commercialisé sous la dénomination de "Huile de Purcellin liquide®" par la Société Dragoco | 3,0 g |
| - Monostéarate de polyéthylèneglycol à 8 moles d'oxyde d'éthylène commercialisé sous la dénomination de "Myrj 45®" par la Société ICI | 3,1 g |
| - Oléate de sorbitane commercialisé sous la dénomination de "Span 80®" par la Société ICI | 1,9 g |
| - Parfums | 0,4 g |

| *Phase B :* | |
|---|---|
| - Pseudolatex de l'exemple II | 26,3 g |
| - Conservateurs | 0,5 g |
| - Eau q.s.p. | 100 g |

### EXEMPLE 7 : Crème de soin

Selon le même mode opératoire que décrit à l'exemple 5, on prépare une crème ayant la composition suivante :

| *Phase A :* | |
|---|---|
| - Ether de 2-éthyl hexyle et de palmitate de glycéryle commercialisé sous la dénomination de "Mexanyl GP®" par la Société Chimex | 12,0 g |
| - Huile de tournesol | 3,0 g |
| - Beurre de karité liquide commercialisé sous la dénomination de "Lipex 202®" par la Société Karlshamns | 5,0 g |
| - Monostéarate de polyéthylèneglycol à 40 moles d'oxyde d'éthylène commercialisé sous la dénomination de "Myrj 52®" par la Société ICI | 1,5 g |
| - Alcool cétylique | 3,5 g |
| - Parfums | 0,7 g |

| *Phase B :* | |
|---|---|
| - Pseudolatex de l'exemple II | 27,4 g |
| - Conservateurs | 0,5 g |
| - Eau q.s.p. | 100 g |

### EXEMPLE 8 : Crème de soin

Selon le même mode opératoire que décrit à l'exemple 5, on prépare une crème ayant la composition suivante :

| *Phase A :* | |
|---|---|
| - Ether de 2-éthyl hexyle et de palmitate de glycéryle commercialisé sous la dénomination de "Mexanyl GP®" par la Société Chimex | 10,00 g |
| - Isostéarate d'isopropyle | 6,00 g |
| - Huile de vaseline | 4,00 g |
| - Monostéarate de polyéthylèneglycol à 40 moles d'oxyde d'éthylène commercialisé sous la dénomination de "Myrj 52®" par la Société ICI | 3,21 g |
| - Laurate de sorbitane commercialisé sous la dénomination de "Span 20®" par la Société ICI | 1,79 g |
| - Parfums | 0,40 g |

| *Phase B :* | |
|---|---|
| - Pseudolatex de l'exemple I | 27,15 g |
| - Conservateurs | 0,30 g |
| - Eau q.s.p. | 100 g |

### EXEMPLE 9 : Crème de soin

| *Phase A :* | |
|---|---|
| - Homopolymère de l'[(acrylamidométhyl-4' benzylidène-3] d1-camphre décrit à l'exemple 1 de FR 86.00274 | 3,0 g |
| - Ether de 2-éthyl hexyle et de palmitate de glycéryle commercialisé sous la dénomination de "Mexanyl GP®" par la Société Chimex | 12,0 g |
| - Isostéarate de glycéryle | 8,0 g |
| - Mélange de stéarate de glycéryle et de monostéarate de polyéthylèneglycol à 100 moles d'oxyde d'éthylène commercialisé sous la dénomination de "Arlacel 165®" par la Société ICI | 2,5 g |
| - Ether d'alcool stéarylique et de polyéthylèneglycol à 2 moles d'oxyde d'éthylène commercialisé sous la dénomination de "Brij 72®" par la Société ICI | 2,0 g |
| - Alcool cétylique | 1,5 g |
| - Parfums | 0,6 g |

| *Phase B :* | |
|---|---|
| - Pseudo-latex de l'exemple I | 26,3 g |
| - Conservateurs | 0,3 g |
| - Eau q.s.p. | 100 g |

La phase A est préparée par dissolution de l'homopolymère de l'[(acrylamidométhyl-4' benzylidène)-3]d1-camphre dans le "Mexanyl GP®" à 80°C, puis refroidissement du mélange à 70°C. On ajoute alors les autres constituants de la phase A.

A la phase B chauffée à 70°C, on ajoute sous agitation, la phase A (70°C) obtenue précédemment. On poursuit alors l'agitation jusqu'à refroidissement du mélange à la température ambiante.

On obtient ainsi une émulsion huile-dans-eau stable, fine et homogène.

Cette crème s'applique facilement sur la peau, ne colle pas et résiste à l'eau. Appliquée tous les jours, elle protège la peau des effets nocifs du soleil et ralentit ainsi son vieillissement prématuré.

### EXEMPLE 10 : Crème de jour

Selon le même mode opératoire que décrit à l'exemple 9, on prépare une crème ayant la composition suivante :

| *Phase A :* | |
|---|---|
| - Homopolymère de l'[(acrylamidométhyl-4' benzylidène-3] d1-camphre décrit à l'exemple 1 de FR 86.00274 | 3,0 g |
| - Ether de 2-éthyl hexyle et de palmitate de glycéryle commercialisé sous la dénomination de "Mexanyl GP®" par la Société Chimex | 12,0 g |
| - Huile de tournesol | 3,0 g |
| - Beurre de karité liquide commercialisé sous la dénomination de "Lipex 202®" par la Société Karlshamns | 5,0 g |
| - Monostéarate de polyéthylèneglycol à 40 moles d'oxyde d'éthylène commercialisé sous la dénomination de "Myrj 52®" par la Société ICI | 1,5 g |
| - Alcool cétylique | 3,5 g |
| - Parfums | 0,7 g |

| *Phase B :* | |
|---|---|
| - Pseudolatex de l'exemple II | 27,4 g |
| - Conservateurs | 0,5 g |
| - Eau q.s.p. | 100 g |

## Revendications

1. Composition cosmétique ou dermatologique destinée à protéger la peau ou un substrat kératinique contre les effets indésirés des radiations ultra-violettes, caractérisée par le fait qu'elle contient au moins une dispersion aqueuse de particules solides, de diamètre moyen compris entre 10 et 400 nm, d'un homopolymère-filtre constitué de motifs répétitifs de formule suivante : 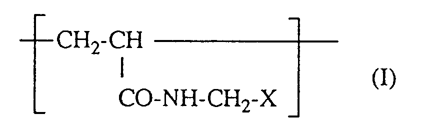 dans laquelle :
X représente un groupement aromatique présentant une absorption ultra-violette dans la zone de longueurs d'onde comprise entre 280 et 400 nm.

2. Composition selon la revendication 1, caractérisée par le fait que X est choisi parmi les groupements de formule : 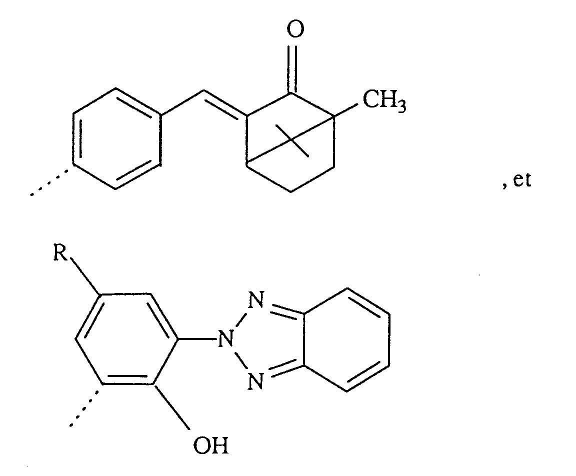 R représentant un groupement tertio-octyle.

3. Composition selon l'une quelconque des revendications précédentes, caractérisée par le fait que l'homopolymère-filtre constituant les particules solides de la dispersion aqueuse a un poids moléculaire inférieur ou égal à 20.000 déterminé par chromatographie d'exclusion stérique.

4. Composition selon l'une quelconque des revendications précédentes, caractérisée par le fait que ladite dispersion aqueuse de particules solides est présente en une proportion telle que la concentration en homopolymère-filtre soit comprise entre 0,5 et 15 % en poids par rapport au poids total de la composition.

5. Composition selon l'une quelconque des revendications précédentes, caractérisée par le fait que ladite dispersion aqueuse de particules solides est un pseudo-latex.

6. Composition selon l'une quelconque des revendications précédentes, caractérisée par le fait qu'elle se présente sous forme d'une solution aqueuse ou hydroalcoolique, d'une lotion, d'une lotion épaissie, d'un gel, d'une crème, d'un lait, d'une émulsion, d'une dispersion vésiculaire ou est conditionnée en aérosol.

7. Composition selon la revendication 6, caractérisée par le fait qu'elle se présente sous forme d'une émulsion, la phase grasse de ladite émulsion contenant, sous forme solubilisée, un homopolymère-filtre de formule (I) tel que défini selon l'une des revendications 1 et 2.

8. Composition selon la revendication 7, caractérisée par le fait que la quantité en homopolymère-filtre de formule (I) présente dans la phase grasse est telle que la proportion totale en homopolymère-filtre dans la composition ne soit pas supérieure à 15 % en poids par rapport au poids total de la composition.

9. Composition selon l'une quelconque des revendications précédentes, caractérisée par le fait qu'elle contient en outre un adjuvant choisi parmi les corps gras, les silicones, les agents épaississants, les agents adoucissants, les filtres solaires UV-A, UV-B, ou à bande large, les agents anti-mousse, les agents hydratants, les parfums, les conservateurs, les agents tensioactifs, les agents plastifiants, les charges, les agents séquestrants, les polymères anioniques, cationiques, non-ioniques, amphotères, ou leurs mélanges, les propulseurs, les agents alcalinisants ou acidifiants, les colorants, et les pigments d'oxydes métalliques.

## Claims

1. Cosmetic or dermatological composition intended for protecting the skin or a keratinous substrate against the undesired effects of ultraviolet radiation, characterized in that it contains at least one agueous dispersion of solid particles, with a mean diameter between 10 and 400 nm, of a homopolymer-screen consisting of repeat units of the following formula: 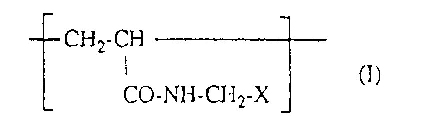 in which:
X represents an aromatic group having an ultraviolet absorption in the wavelength region between 280 and 400 nm.

2. Composition according to Claim 1, characterized in that X is chosen from the groups of formula: 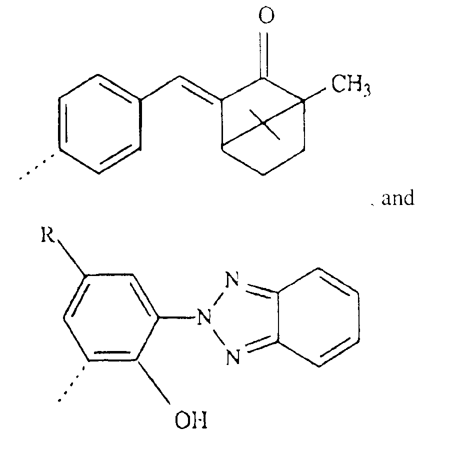 R representing a tert-octyl group.

3. Composition according to either of the preceding claims, characterized in that the homopolymer-screen constituting the solid particles of the aqueous dispersion has a molecular weight less than or equal to 20,000 determined by steric exclusion chromatography.

4. Composition according to any one of the preceding claims, characterized in that the said agueous dispersion of solid particles is present in a proportion such that the concentration of homopolymer-screen is between 0.5 and 15% by weight with respect to the total weight of the composition.

5. Composition according to any one of the preceding claims, characterized in that the said agueous dispersion of solid particles is a pseudolatex.

6. Composition according to any one of the preceding claims, characterized in that it is provided in the form of an agueous or agueous/alcoholic solution, of a lotion, of a thickened lotion, of a gel, of a cream, of a milk, of an emulsion or of a vesicular dispersion or is packaged as an aerosol.

7. Composition according to Claim 6, characterized in that it is provided in the form of an emulsion, the fatty phase of the said emulsion containing, in the dissolved form, a homopolymer-screen of formula (I) as defined according to either of Claims 1 and 2.

8. Composition according to Claim 7, characterized in that the amount of homopolymer-screen of formula (I) present in the fatty phase is such that the total proportion of homopolymer-screen in the composition is not greater than 15% by weight with respect to the total weight of the composition.

9. Composition according to any one of the preceding claims, characterized in that it additionally contains an adjuvant chosen from fatty substances, silicones, thickening agents, emollients, UV-A, UV-B or broad spectrum sunscreens, antifoaming agents, moisturizing agents, fragrances, preserving agents, surface-active agents, plasticizers, fillers, sequestering agents, anionic, cationic, nonionic or amphoteric polymers or their mixtures, propellants, basifying or acidifying agents, dyes and metal oxide pigments.

## Patentansprüche

1. Kosmetische oder dermatologische Zubereitung, die zum Schutz der Haut oder eines Keratinsubstrats gegen unerwünschte Wirkungen ultravioletter Bestrahlung bestimmt ist, dadurch gekennzeichnet, daß sie mindestens eine wäßrige Dispersion von festen Teilchen mit einem mittleren Durchmesser zwischen 10 und 400 nm eines Homopolymerfilters enthält, die aus wiederkehrenden Einheiten der folgenden Formel: 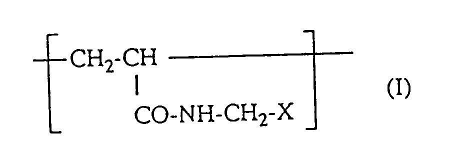 bestehen, worin:
X eine aromatische Gruppe darstellt, die eine UV-Absorption im Wellenlängenbereich zwischen 280 und 400 nm aufweist.

2. Zubereitung gemäß Anspruch 1, dadurch gekennzeichnet, daß X aus den Gruppen der Formeln: 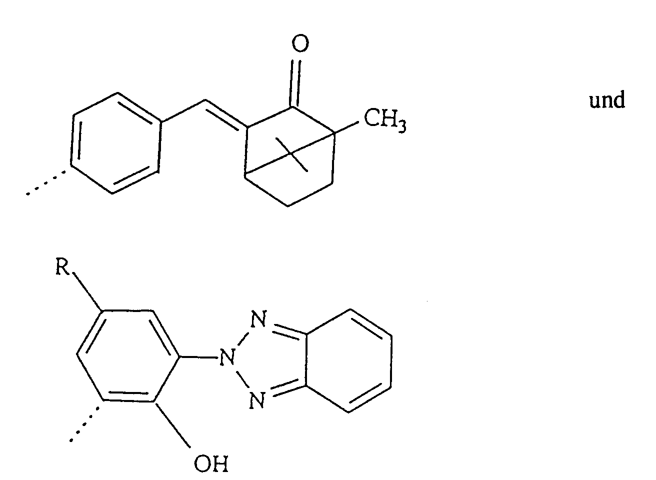 gewählt ist,
worin R für eine tert.-Octylgruppe steht.

3. Zubereitung gemäß einem der vorstehenden Ansprüche, dadurch gekennzeichnet, daß der aus festen Teilchen bestehende Homopolymerfilter der wäßrigen Dispersion ein Molekulargewicht kleiner oder gleich 20.000 aufweist, welches mittels sterischer Ausschlußchromatographie bestimmt ist.

4. Zubereitung gemäß einem der vorstehenden Ansprüche, dadurch gekennzeichnet, daß die wäßrige Dispersion der festen Teilchen in einem solchen Anteil vorliegt, daß die Konzentration an Homopolymerfilter zwischen 0,5 und 15 Gew.-%, bezogen auf das Gesamtgewicht der Zubereitung, liegt.

5. Zubereitung gemäß einem der vorstehenden Ansprüche, dadurch gekennzeichnet, daß die wäßrige Dispersion der festen Teilchen ein Pseudo-Latex ist.

6. Zubereitung gemäß einem der vorstehenden Ansprüche, dadurch gekennzeichnet, daß sie in Form einer wäßrigen oder hydroalkoholischen Lösung, einer Lotion, einer verdickten Lotion, eines Gels, einer Creme, einer Milch, einer Emulsion, einer vesiculären Dispersion vorliegt oder als Aerosol konditioniert ist.

7. Zubereitung gemäß Anspruch 6, dadurch gekennzeichnet, daR sie in Form einer Emulsion vorliegt, wobei die Fettphase der Emulsion in solubilisierter Form einen Homopolymerfilter der Formel (I) gemäß einem der Ansprüche 1 und 2 enthält.

8. Zubereitung gemäß Anspruch 7, dadurch gekennzeichnet, daß die Menge an Homopolymerfilter der Formel (I) in der Fettphase so beschaffen ist, daß der Gesamtanteil an Homopolymerfilter in der Zubereitung 15 Gew.-%, bezogen auf das Gesamtgewicht der Zubereitung, nicht übersteigt.

9. Zubereitung gemäß einem der vorstehenden Ansprüche, dadurch gekennzeichnet, daß sie zusätzlich ein Adjuvans enthält, welches ausgewählt ist aus Fettkörpern, Silikonen, Verdickungsmitteln, Enthärtern, UV-A-, UV-B- oder Breitband-Sonnenfiltern, Schaumbremsern, Feuchthaltemitteln, Parfümen, Konservierungsmitteln, oberflächenaktiven Mitteln (Tensiden), Weichmachern, Zuschlägen, Sequestrierungsmitteln, anionischen, kationischen, nichtionischen oder amphoteren Poly-meren oder deren Mischungen, Treibmitteln, Alkalinisierung- oder Acidifizierungsmitteln, Farbstoffen und Pigmenten von Metalloxiden.
